# EUROPÄISCHE PATENTSCHRIFT

(11) **EP 1 078 912 B1**
(45) Veröffentlichungstag und Bekanntmachung des Hinweises auf die Patenterteilung: **08.01.2003**
(21) Anmeldenummer: 00116254.4
(22) Anmeldetag: 08.08.2000
(51) Int. Cl.: C07C 67/303, C07C 69/608

(54) **Verfahren zur Herstellung von Methyl Cyclohexylpropionat**
Process for the preparation of methyl cyclohexylpropionate
Procédé de préparation du cyclohexylepropionate de méthyle

(30) Priorität: 20.08.1999 DE 19939544
(43) Veröffentlichungstag der Anmeldung: 28.02.2001
(73) Patentinhaber: HAARMANN & REIMER GMBH, 37601 Holzminden (DE)
(72) Erfinder: Kuhn, Walter, Dr., 37603 Holzminden (DE); Funk, Hans-Ulrich, 37697 Lauenförde (DE); Senft, Gerhard, 37603 Holzminden (DE); Kiel, Wolfgang, Dr., 51519 Odenthal (DE)
(74) Vertreter: Mann, Volker, Dr.

(56) Entgegenhaltungen:
- US-A- 5 286 898
- US-A- 5 319 129
- CHEMICAL ABSTRACTS, vol. 132, no. 22, 29. Mai 2000 (2000-05-29) Columbus, Ohio, US; abstract no. 293517t, CERVENY LIBOR ET AL,: "The Synthesis of Allyl-3-Cyclohexylpropionate" Seite 667; Spalte r; XP002158953 & CERVENY LIBOR ET AL.: PERFUM.FLAVOR, Bd. 25, Nr. 1, 2000, Seiten 19-23,

## Beschreibung

Die Erfindung betrifft ein Verfahren zur Herstellung von Methyl-cyclohexyl-propionat in Gegenwart eines Ruthenium- und/oder Palladiumkatalysators ausgehend von Methylcinnamat.

In C. R. Hebd. Seances Acad. Sci. 1913, 156, 751 bis 753 ist die Hydrierung von Methylcinnamat zu Methyl-cyclohexyl-propionat unter Raney-Nickel-Katalyse bei 170 bis 185°C beschrieben. Bei Erhöhung der Temperatur auf 200°C kommt es zur Esterspaltung und Inaktivierung des Raney-Nickel-Katalysators. Nach dem Verfahren entsteht Methyl-cyclohexyl-propionat mit nicht ausreichender Reinheit.

Aufgabe der vorliegenden Erfindung war es, Methyl-cyclohexyl-propionat in großer Reinheit herzustellen.

Es wurde ein Verfahren zur Herstellung von Methyl-cyclohexyl-propionat durch Hydrierung von Methylcinnamat in Gegenwart eines Katalysators gefunden, das dadurch gekennzeichnet ist, dass ein Ruthenium- und/oder Palladiumkatalysator eingesetzt wird.

Ruthenium- und Palladiumkatalysatoren sind an sich bekannt (Methoden der organischen Chemie/Houben Weyl, Band IV/1c, Reduktion Teil 1, Georg Thieme Verlag, Stuttgart, 1980, Seiten 15 bis 562, Handbook of Heterogeneous Catalysis, Vol. 1-5, Seiten 2123 bis 2447; VCH Weinheim; 1997).

Die Katalysatoren werden in der Regel auf einen Träger aufgebracht. Als Träger kommen beispielsweise Aktivkohle, Siliciumdioxid, Calciumcarbonat oder Aluminiumoxid in Frage. Der bevorzugte Träger ist Aktivkohle.

Der Träger für das erfindungsgemäße Verfahren enthält im allgemeinen 1 bis 20 Gew.-%, bevorzugt 5 bis 10 Gew.-%, Ruthenium und/oder Palladium bezogen auf den Gesamtkatalysator.

Es können auch Mischkatalysatoren aus Ruthenium und Palladium eingesetzt werden.

Für das erfindungsgemäße Verfahren kann der Katalysator im trockenen oder feuchten Zustand (bis zu 60 % Wasser) verwendet werden.

Für das erfindungsgemäße Verfahren beträgt das Gewichtsverhältnis des eingesetzten Katalysators zu Methylcinnamat 0,0001 bis 0,1 zu 1, bevorzugt 0,005 bis 0,05 zu 1.

Die Reaktionstemperatur für das erfindungsgemäße Verfahren liegt bei 30 bis 250°C, bevorzugt bei 60 bis 200°C.

Der Wasserstoffdruck liegt bei 1 bis 100 bar, bevorzugt bei 10 bis 20 bar.

Die Reaktionszeit beträgt 2 bis 100 Stunden, bevorzugt 5 bis 40 Stunden.

Das erfindungsgemäße Verfahren kann durch die folgende Reaktionsgleichung dargestellt werden:

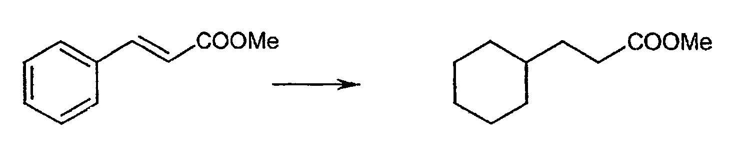

Das erfindungsgemäße Verfahren wird im allgemeinen wie folgt durchgeführt:

In einem Druckbehälter mit Rührer wird das Methylcinnamat und der Katalysator vorgelegt. Es wird bei der gewählten Reaktionstemperatur und Wasserstoffdruck hydriert. Das so erhaltene Methyl-cyclohexyl-propionat wird nach Entfernen des Katalysators durch Filtration, Dekantieren oder Zentrifugation erhalten.

Nach dem erfindungsgemäßen Verfahren fällt Methyl-cyclohexyl-propionat in einer Reinheit von >99,5 % und einer Ausbeute von etwa 99 % an.

Methyl-cyclohexyl-propionat ist ein Riechstoff mit fruchtigem Charakter, der gleichzeitig auch Ausgangsstoff zur Herstellung von Allylcyclohexylpropionat ist. Allylcyclohexylpropionat ist ein Riechstoff mit starkem Ananasgeruch (S. Arctander, Perfume and Flavour Chemicals, No. 77,1969).

Methylcinnamat für das erfindungsgemäße Verfahren kann durch Veresterung von Zimtsäure mit Methanol oder durch Claisen-Kondensation von Benzaldehyd und Methylacetat hergestellt werden (K. Bauer, D. Garbe, Fragrance and Flavour Materials, Seite 80, Weinheim, VCH Verlagsgesellschaft, 1985).

### Beispiele

### Beispiel 1

In einen 51 Rührautoklaven mit Begasungsrührer werden 1565 g Methylcinnamat und 10,3 g 5 gew.-%iges Ruthenium auf Aktivkohle feucht vorgelegt. Es wird 6 Stunden bei 155°C und 20 bar hydriert. Nach Filtration werden 1625 g Methyl-cyclohexyl-propionat mit einer Reinheit von 99,6 Gew.-% erhalten. Das erhaltene Methyl-cyclohexyl-propionat kann ohne Rückstand bei 135°C Sumpftemperatur und 35 mbar Vakuum destilliert werden. Die Ausbeute d. Th. beträgt 99 %.

### Beispiel 2

In einen 5 Rührautoklaven mit Begasungsrührer werden 2000 g Methylcinnamat und 19 g 5 gew.-%iges Palladium auf Aktivkohle feucht vorgelegt. Es wird 35 Stunden bei 160 bis 170°C und 20 bar hydriert. Nach Filtration werden 2050 g Methyl-cyclohexyl-propionat mit einer Reinheit von 99,7 % erhalten. Das erhaltene Methyl-cyclohexyl-propionat kann ohne Rückstand bei 135°C Sumpftemperatur und 35 mbar Vakuum destilliert werden. Die Ausbeute d. Th. beträgt 98 %.

## Patentansprüche

1. Verfahren zur Herstellung von Methyl-cyclohexyl-propionat durch Hydrierung von Methylcinnamat in Gegenwart eines Katalysators, **dadurch gekennzeichnet, dass** ein Ruthenium- und/oder Palladiumkatalysator eingesetzt wird.

2. Verfahren nach Anspruch 1, **dadurch gekennzeichnet, dass** das Gewichtsverhältnis des eingesetzten Katalysators zu Methylcinnamat 0,0001 bis 0,1 zu 1 beträgt.

3. Verfahren nach den Ansprüchen 1 und 2, **dadurch gekennzeichnet, dass** die Reaktionstemperatur im Bereich von 30 bis 250°C liegt.

4. Verfahren nach den Ansprüchen 1 bis 3, **dadurch gekennzeichnet, dass** das Verfahren bei einem Wasserstoffdruck von 1 bis 100 bar durchgeführt wird.

## Claims

1. Process for the preparation of methyl cyclohexylpropionate by hydrogenation of methyl cinnamate in the presence of a catalyst, **characterised in that** a ruthenium and/or palladium catalyst is used.

2. Process according to claim 1, **characterised in that** the ratio by weight of the catalyst used to methyl cinnamate is between 0.0001 and 0.1 to 1.

3. Process according to claims 1 and 2, **characterised in that** the reaction temperature is in the range of 30 to 250°C.

4. Process according to claims 1 to 3, **characterised in that** the process is carried out at a hydrogen pressure of 1 to 100 bar.

## Revendications

1. Procédé pour la préparation de propionate de méthylcyclohexyle par hydrogénation de cinnamate de méthyle en présence d'un catalyseur, **caractérisé en ce que** l'on utilise un catalyseur de ruthénium et/ou de palladium.

2. Procédé selon la revendication 1, **caractérisé en ce que** le rapport massique du catalyseur utilisé au cinnamate de méthyle est de 0,0001 à 0,1 par rapport à 1.

3. Procédé selon les revendications 1 et 2, **caractérisé en ce que** la température de la réaction se trouve dans le domaine de 30 à 250°C.

4. Procédé selon les revendications 1 à 3, **caractérisé en ce que** l'on réalise le procédé à une pression d'hydrogène de 1 à 100 bars.
